(19) **Europäisches Patentamt**

**European Patent Office**

**Office européen des brevets**

(11) Veröffentlichungsnummer: **0 339 331**
**A2**

(12) **EUROPÄISCHE PATENTANMELDUNG**

(21) Anmeldenummer: **89106132.7**

(22) Anmeldetag: **07.04.89**

(51) Int. Cl.⁴: **C12Q 1/58 , G01N 33/52 ,
G01N 33/04**

(30) Priorität: **29.04.88 DD 315210**

(43) Veröffentlichungstag der Anmeldung:
**02.11.89 Patentblatt 89/44**

(84) Benannte Vertragsstaaten:
**BE CH DE FR GB LI LU NL SE**

(71) Anmelder: **VEB Arzneimittelwerk Dresden
Wilhelm-Pieck-Strasse 35
DDR-8122 Radebeul 1(DD)**

(72) Erfinder: **Harseim, Bärbel
Werrastrasse 12
DDR-5901 Ebenshausen(DD)**
Erfinder: **Hartung, Horst, Dr.
Karlstrasse 17
DDR-5900 Eisenach(DD)**
Erfinder: **Reichardt, Werner, Dr.
Ludwigstrasse 23
DDR-5900 Eisenach(DD)**
Erfinder: **Winkler, Manfred, Dr.
Amtshainersdorfer Siedlung 40
DDR-8360 Sebnitz(DD)**
Erfinder: **Kallies, Karl-Heinz
Götzinger Strasse 17
DDR-8360 Sebnitz(DD)**

(74) Vertreter: **Puchberger, Rolf, Dipl. Ing. et al
Patentanwälte, Dipl. Ing. Georg Puchberger
Dipl. Ing. Rolf Puchberger Dipl. Ing. Peter
Puchberger Singerstrasse 13 Postfach 55
A-1010 Wien(AT)**

(54) **Teststreifen zur Bestimmung von Harnstoff und seine Anwendung.**

㉗ Die Erfindung betrifft einen Teststreifen zur quantitativen Bestimmung von Harnstoff, insbesondere in Milch. Es handelt sich um einen ökonomischen und schnellen Test, der große Serienuntersuchungen auch in landwirtschaftlichen Praxisbetrieben erlaubt, ohne zusätzliche Apparaturen oder Meßgeräte zu benötigen. Die Bestimmung erfolgt mit Hilfe eines Teststreifens, dessen Reaktionszone mit einem aus Urease, Glukose, Base und Tensid bestehenden Reaktionssystem imprägniert ist, wobei durch Vergleich mit einer Eichtabelle oder nach einer Berechnungsformel, bezogen auf einen wäßrigen Harnstoffstandard, die Ermittlung der Harnstoffkonzentration in einfacher Weise erfolgt.

EP 0 339 331 A2

## Teststreifen zur Bestimmung von Harnstoff und seine Anwendung

Anwendungsgebiet der Erfindung

Die Erfindung betrifft einen Teststreifen und seine Verwendung zur schnellen quantitativen Bestimmung des Harnstoffgehaltes, vorzugsweise von Milch. Er kann sowohl in der Landwirtschaft, als auch in der miichverarbeitenden Industrie unmittelbar angewendet werden.

Charakteristik des bekannten Standes der Technik

Für die quantitative Bestimmung des Harnstoffgehaltes von Milch sind verschiedene Methoden bekannt, z.B. die enzymatische Spaltung des Harnstoffes mit Urease und die titrimetrische Bestimmung des hierbei gebildeten Ammoniaks, der kolorimetrischen Messung eines durch Umsetzung des Harnstoffs mit Phenol und Alkalihypochlorit gebildeten blauen Farbstoffes (Thaler, H. und Sturm, W., Deut. Lebensm. Rundsch. 61 (1966) 35 - 40; Krom, M. D., Analyst 105 (1980) 305 - 316; Broderick, G. A., Kang, J. H., Linow, F. und Schaffner, B., Nahrung 24 (1980) 85 - 87; Behringer, G. und Klostermeyer, H. J., Lebensmittel Unters. Forsch. 172 (1981) 362 - 364) oder die Farbreaktionen zwischen Harnstoff und p-Dimethylaminobenzaldehyd (Brown, H. H., Anal. Chem. 31 (1959) 1844 - 1846; McDowell, A. K. R., J. Dairy Res. 39 (1972) 27 - 33) bzw. Diacetylmonoxim (Vignon, B., Dissertation, Université Nancy I (1976); Siast, G., Ann. Biol. Clin. 26 (1968) 431 - 448). Die von der Firma Boehringer vorgeschlagene Schnellbestimmung basiert auf der Spaltung des Harnstoffs durch das Enzym Urease und der anschließenden Umsetzung des dabei gebildeten Ammoniaks mit alpha-Ketoglutarat in Gegenwart von Glutamat-Dehydrogenase, wobei die bei der Umsetzung verbrauchte Menge NaOH spektralphotometrisch bestimmt wird.

Der Einsatz dieser Analysenverfahren setzt jedoch aufwendige und zeitraubende Laboranalysen voraus, die eine schnelle Ermittlung des Harnstoffgehaltes beispielsweise in landwirtschaftlichen Praxisbetrieben unmöglich machen.

Das von der Firma Boehringer entwickelte Teststreifenverfahren zur Ermittlung des Harnstoffgehaltes in Blut, Serum und Plasma basiert auf einer Auswertung der Farbreaktion und erfordert die Anwendung eines Reflexionsphotometers zur Bestimmung der Farbintensität, die der Harnstoffkonzentration proportional ist.

Der von der Firma Merck Darmstadt vertriebene Teststreifen für Harnstoff gestattet die Bestimmung des Harnstoffgehaltes in Blut, versagt aber bei Milch.

Gleiches gilt auch für die Harnstoffbestimmung nach der DD-PS 137 384. Auf Grund der hohen Pufferwirkung der Milch wird der durch das Enzym Urease gebildete Ammoniak in der Reaktionszone festgehalten und diffundiert nicht in die Indikatorschicht. Teilweise verhindern auch Benetzungsschwierigkeiten zwischen Milch und Teststreifen einen ordnungsgemäßen Nachweis.

Ziel der Erfindung

Ziel der Erfingung ist es, ein ökonomisches und rasches Verfahren zur quantitativen Bestimmung des Harnstoffgehaltes von Milch zur entwickeln, das mit Hilfe eines geeigneten Teststreifens die Nachteile bekannter Verfahren beseitigt.

Darlegung des Wesens der Erfindung

Der Erfindung liegt die Aufgabe zugrunde, einen ökonomischen und schnellen Test zur quantitativen Bestimmung des Harnstoffgehaltes von Milch zu entwickeln, der unter den Bedingungen der landwirtschaftlichen Tierhaltung eingesetzt werden kann. Die Aufgabe der Erfindung liegt auch darin begründet, die Freisetzung des Ammoniaks aus der Reaktionszone zu erreichen, ohne die Aktivität des Enzyms Urease wesentlich zu beeinträchtigen.

Die Aufgabe wird erfindungsgemäß dadurch gelöst, daß der Harnstoffgehalt der Milchproben mit einem Harnstoff-Teststreifen bestimmt wird, der aus einer Reaktionszone und einer Indikatorzone besteht, die auf einem Träger aufgebracht sind. Es wurde gefunden, daß durch die Imprägnierung eines saugfähigen Trägermaterials mit Urease-Enzym, Glukose, einer Base und einem Tensid eine Reaktionszone entsteht, die eine enzymatische Spaltung des Harnstoffs aus der darauf gebrachten Milch bewirkt.

Das Trägermaterial der Reaktionszone kann aus natürlichen oder auch synthetischen Fasern bestehen, wobei das Trägermaterial keinen Einfluß auf die Umsetzung haben darf und genügend saugfähig sein soll. Vorteilhaft wird Filterpapier verwendet, dessen Gewicht zwischen 60 und 400 g/m² liegt.

Als Basen für den Aufbau der Reaktionszone sind Natronlauge, Kalilauge, vorteilhaft aber Salze schwacher Säuren, wie Kaliumkarbonat, Natriumkarbonat, Magnesiumkarbonat oder Natriumcitrat, einsetzbar. Die Base soll nicht zu stark sein (Laugen), damit das Enzym nicht zerstört wird bzw. das System den optimalen pH-Bereich verläßt. Andererseits muß natürlich die Milch noch abgepuffert werden, damit Ammoniak überhaupt ausgetrieben werden kann.

Es hat sich gezeigt, daß durch den Einsatz von nichtionogenen Tensiden, wie etwa Alkylphenylpolyethylenglykol oder Alkylpolyglykolethersulfat, bzw. von Aniontensiden, wie z.B. Alkylsulfaten, eine gute Lösung der in der Milch vorhandenen Proteine und des Calziums erreicht wird. Es kommt zu einer guten Benetzbarkeit der verfestigten Reaktionszone mit der Probeflüssigkeit Milch, während bei einem Einsatz von kationischen Tensiden diese Benetzbarkeit schlechter erreicht wird.

Auf der Reaktionszone ist die Imprägnierung dabei in folgenden Konzentrationen aufgebracht:

Urease in einer Konzentration von 0,30 bis 0,90 mg/cm², vorzugsweise 0,70 mg/cm²,

Glukose in einer Konzentration von 0,05 bis 0,30 mg/cm², vorzugsweise 0,17 mg/cm²,

Tensid in einer Konzentration von 0,25 bis 1,30 mg/cm², vorzugsweise 0,63 mg/cm²,

Base in einer Konzentration von 0,2 bis 0,4 mg/cm², vorzugsweise 0,28 mg/cm².

Durch den Einsatz einer genannten Base in dem bezeichneten Konzentrationsbereich wird die Aktivität der Urease nicht beeinträchtigt. Es gelingt so aber die Freisetzung des Ammoniaks aus den Salzen der Milch und die Diffusion in die Indikatorzone.

Der Zusatz eines erfindungsgemäßen Tensides ermöglicht die Benetzbarkeit der verfestigten Reaktionszone, so daß definierte Mengen der Probeflüssigkeit aufgenommen werden.

Auf einer Unterlage, die aus einem hydrophoben Material, vorteilhaft aus wasserundurchlässigem Plastmaterial, besteht, ist die so gewonnene Reaktionszone an einem Ende und daran anschließend eine auf Ammoniak ansprechende, in bekannter Weise gewonnene Indikatorzone angeordnet. Die Abmessungen betragen vorteilhaft für den Träger (Unterlage) 70 x 6 mm, die Reaktionszone 6 x 6 mm, die Indikatorzone 40 x 6 mm, wobei der Abstand von Reaktions- zu Indikatorzone 5 mm beträgt. Reaktions- und Indikatorzone sind auf der Unterlage mit einem wasserunlöslichen Kleber fixiert. Ein solcher Teststreifen befindet sich zum Zwecke des Nachweises in einem einseitig geschlossenen Röhrchen, wobei die Reaktionszone dem geschlossenen Ende anliegt.

Der Vorteil des erfindungsgemäßen Teststreifens besteht in der einfachen Handhabung auch unter den Bedingungen der landwirtschaftlichen Praxis ohne Anwendung spezieller Apparate oder Meßgeräte.

Die Bestimmungen werden so durchgeführt, daß der Teststreifen mit der Reaktionszone für 3 Sekunden in die zu untersuchende Milchprobe eingetaucht oder 15 μl der Probelösung aufpipettiert werden. Wesentlich ist, daß mit dieser Handhabung auf die Reaktionszone die zu untersuchende Probe in einer Menge von 0,040 bis 0,070 ml/cm², vorzugsweise 0,056 ml/cm², dosiert wird. Anschließend bringt man den Streifen in ein einseitig geschlossenes Glasröhrchen derart, daß sich die Reaktionszone am Boden des waagerecht liegenden Röhrchens befindet. Nach 120 Minuten mißt man die Länge der Verfärbung der Indikatorzone, die der in der Milchprobe vorhandenen Harnstoffmenge proportional ist. Die Bestimmung der Harnstoffkonzentration erfolgt einmal durch Vergleich der Längen der gefärbten Indikatorzonen mit den Werten einer nach einer anderen Methode erstellten Eichtabelle.

Zum anderen ist es möglich, neben der durch eine Probe gefärbten Indikatorzone eine Indikatorzone mit einer Harnstofflösung bekannter Konzentration (z.B. 10 mg/100 ml) in Wasser zu behandeln. Durch Ausmessen der gefärbten Abschnitte der Indikatorzonen erhält man die beiden Größen x = Länge Abschnitt Probe,

y = Länge Abschnitt Standard,

und errechnet die Harnstoffkonzentration nach den Formeln

$$\frac{\text{mg Harnstoff}}{100 \text{ ml}} = \frac{x \cdot \text{Konzentration Standard}}{y \cdot 0,76} \qquad\qquad \text{I}$$

$$\frac{\text{mmol Harnstoff}}{1\ 000 \text{ ml}} = \frac{x \cdot \text{Konzentration Standard}}{y} \cdot 7,89 \qquad \text{II}$$

Mit Beispielen soll das erfindungsgemäße Testmaterial und seine Verwendung näher erläutert werden.

Ausführungsbeispiele

Beispiel 1

In ein Glasgefäß mit den Maßen 300 x 40 x 30 mm werden 200 ml einer ethanolischen Lösung von 0,161 g Weinsäure und 100 ml einer ethanolischen Lösung von 0,256 g Bromkresolgrün gegeben. Mit dieser Lösung imprägniert man durch Eintauchen (1 Minute), Lufttrocknung und anschließende Trocknung im Trockenschrank bei 70° C für eine Stunde 37 Papierstreifen mit den Maßen 300 x 40 mm, aus denen durch Schneiden von Streifen zu je 6 mm Breite und 40 mm Länge die Indikatorzonen hergestellt werden.

In ein Glasgefäß werden 75 ml einer wäßrigen Lösung von 1,2 g Urease-Enzym und 0,3 g Glukose sowie 0,450 ml einer gesättigten Kaliumkarbonatlösung in Wasser sowie 1,12 g Alkylpolyglykolethersulfat gegeben. Mit dieser Lösung imprägniert man durch Eintauchen (1 Minute) und Lufttrocknung 15 Papierstreifen mit den Maßen 300 x 40 mm. Nach Schneiden von Papierstücken der Maße 6 x 6 mm erhält man die fertigen Reaktionszonen.

Auf einen wasserundurchlässigen Plaststreifen mit den Maßen 70 x 6 mm klebt man je ein Stück Reaktionszone und Indikatorzone dergestalt, daß sich am Ende des Streifens die Reaktionszone und in einem Abstand von 5 mm davon die Indukatorzone befindet. Es ist darauf zu achten, daß mit einem wasserunlöslichen Kleber auf Kautschukbasis geklebt wird.

In eine Milchprobe taucht man den Teststreifen ca. 3 Sekunden bis zur vollständigen Benetzung der Reaktionszone ein. Die überschüssige Flüssigkeit streift man ab. Anschließend wird der Streifen in ein einseitig geschlossenes Glasröhrchen mit einem Durchmesser von 6,5 mm so eingeschoben, daß sich die Reaktionszone am geschlossenen Ende befindet. Nach 120 Minuten wird die Länge der gefärbten Indikatorzone ausgemessen. Aus einer Eichtabelle wird der Wert der Harnstoffkonzentration ermittelt. Die Aufstellung der Eichtabelle erfolgt gegen eine andere Harnstoffmethode (z.B. Conway-Bestimmung). Die Eichtabelle ist für eine komplette Charge an Teststreifen gültig.

Beispiel 2

Auf die Reaktionszone eines nach Beispiel 1 hergestellten Teststreifens werden 15 $\mu$l Milch aufpipettiert. Der Teststreifen wird wie im Beispiel 1 beschrieben in ein Glasröhrchen geschoben. Analog behandelt man zwei Harnstofflösungen bekannter Konzentration (20 mg/100 ml und 30 mg/100 ml) in Wasser. Durch Ausmessen der gefärbten Abschnitte der Indikatorzonen erhält man die beiden in die Formel I bzw. II einzusetzenden Größen x und y.

Im Falle einer Milch mit einem Harnstoffgehalt von 25 mg/100 ml ergeben sich nach 120 Minuten Reaktionszeit folgende Meßwerte:

$$\text{Milch:} \qquad x = 8,5 \text{ mm}$$

$$20 \text{ mg/100 ml:} \quad y = 9,5 \text{ mm} \;) \text{ Durchschnitt:}$$

$$30 \text{ mg/100 ml:} \quad y = 13,0 \text{ mm} \;) \; 25 \text{ mg/100 ml:} \; y = 11,25 \text{ mm}$$

$$\frac{\text{mg Harnstoff}}{100 \text{ ml}} = \frac{8,5 \cdot 25}{11,25 \cdot 0,76} = \underline{\underline{24,85}}$$

**Ansprüche**

1. Teststreifen zur Bestimmung von Harnstoff mittels Urease, auf dessen in einem Röhrchen befindlichen Träger eine Reaktions- und eine Indikatorzone angeordnet sind, dadurch gekennzeichnet, daß auf einem in einem einseitig geschlossenen Röhrchen befindlichen hydrophoben Träger neben einer an sich bekannten, im sauren Bereich umschlagenden Indikatorzone eine Reaktionszone mit einem aus Urease, Glukose, Base und nichtionogenem Tensid bzw. Aniontensid bestehenden Reaktionssystem angeordnet ist.

4

2. Teststreifen nach Anspruch 1, dadurch gekennzeichnet, daß die Reaktionszone eine Konzentration für Urease von 0,30 bis 0,90 mg/cm², vorzugsweise 0,70 mg/cm²,
Glukose von 0,05 bis 0,3 mg/cm², vorzugsweise 0,17 mg/cm²,
Base von 0,2 bis 0,4 mg/cm², vorzugsweise 0,28 mg/cm²,
Tensid von 0,25 bis 1,30 mg/cm², vorzugsweise 0,63 mg/cm²,
enthält.

3. Teststreifen nach den Ansprüchen 1 und 2, dadurch gekennzeichnet, daß das Reaktionssystem als Base Natronlauge, Kalilauge, vorzugsweise jedoch Salze schwacher Säuren, wie Kaliumkarbonat, Natriumkarbonat, Magnesiumkarbonat oder Natriumcitrat, enthält.

4. Teststreifen nach den Ansprüchen 1 und 2, dadurch gekennzeichent, daß das Reaktionssystem als nichtionogenes Tensid Alkylphenylpolyethylenglykol oder Alkylpolyglykolethersulfat enthält.

5. Teststreifen nach den Ansprüchen 1 und 2, dadurch gekennzeichnet, daß das Reaktionssystem als Aniontensid Alkylsulfate enthält.

6. Teststreifen nach den Ansprüchen 1 bis 5, dadurch gekennzeichnet, daß die Reaktionszone am geschlossenen Ende des Röhrchens angeordnet ist.

7. Teststreifen nach den Ansprüchen 1 bis 6, dadurch gekennzeichnet, daß die Reaktionszone und die Indikatorzone mit einem hydrophoben Träger mittels wasserunlöslichem Kleber verbunden sind.

8. Teststreifen nach den Ansprüchen 1 bis 7, dadurch gekennzeichnet, daß der Träger die Abmessungen 70 x 6 mm, die Reaktionszone 6 x 6 mm und die Indikatorzone 40 x 6 mm besitzt, wobei Reaktions- und Indikatorzone auf dem Träger einen geringen Abstand, vorzugsweise von 5 mm, aufweisen.

9. Verfahren zur Anwendung eines Teststreifens zur Bestimmung von Harnstoff nach den Ansprüchen 1 bis 8, dadurch gekennzeichnet, daß man auf die Reaktionszone eine Probe aufbringt, den Teststreifen mit der Reaktionszone zuerst in ein waagerecht liegendes, einseitig geschlossenes Röhrchen einbringt, die Färbung der Indikatorzone ausmißt und die Konzentration des Harnstoffs mit einem Komparator oder im Vergleich zu einem wäßrigen Harnstoffstandard nach den Formeln

$$\frac{mg\ Harnstoff}{100\ ml} = \frac{x \cdot Konzentration\ Standard}{y \cdot 0,76} \qquad\qquad I$$

$$\frac{mmol\ Harnstoff}{1000\ ml} = \frac{x \cdot Konzentration\ Standard}{y} \cdot 7,89 \qquad II$$

worin x = Länge Abschnitt Probe und
y = Länge Abschnitt Standard
bedeutet, ermittelt.

10. Verfahren nach Anspruch 9, dadurch gekennzeichnet, daß man die Probe in einer Menge von 0,040 bis 0,070 ml/cm², vorzugsweise 0,056 ml/cm², aufbringt.

11. Verfahren nach den Ansprüchen 9 und 10, dadurch gekennzeichnet, daß man die Probe durch Eintauchen der Reaktionszone für 3 Sekunden oder durch Aufpipettieren von ca. 15 µl aufbringt.

12. Verfahren nach den Ansprüchen 9 bis 11, dadurch gekennzeichnet, daß man die Färbung der Indikatorzone nach 120 Minuten ausmißt.

5